# EUROPÄISCHE PATENTSCHRIFT

(11) **EP 1 492 872 B1**
(45) Veröffentlichungstag und Bekanntmachung des Hinweises auf die Patenterteilung: **12.09.2007**
(21) Anmeldenummer: 03720344.5
(22) Anmeldetag: 21.03.2003
(51) Int. Cl.: C12N 9/10, C12N 15/67

(54) **EXPRESSION VON PHOSPHOLIPID:DIACYLGLYCERIN-ACYLTRANSFERASE (PDAT) ZUR HERSTELLUNG VON PFLANZLICHEN SPEICHERLIPIDEN ENTHALTEND MEHRFACH UNGESÄTTIGTE FETTSÄUREN**
EXPRESSION OF PHOSPHOLIPID:DIACYLGLYCERINE ACYLTRANSFERASE (PDAT) FOR THE PRODUCTION OF PLANT STORAGE LIPIDS WITH POLYUNSATURATED FATTY ACIDS
EXPRESSION DE LA PHOSPHOLIPIDE : DIACYLGLYCEROL-ACYLTRANSFERASE (PDAT) POUR LA PRODUCTION DE LIPIDES DE STOCKAGE VEGETAUX CONTENANT DES ACIDES GRAS POLYINSATURES

(30) Priorität: 30.03.2002 DE 10214410
(43) Veröffentlichungstag der Anmeldung: 05.01.2005
(73) Patentinhaber: BASF Plant Science GmbH, 67056 Ludwigshafen (DE)
(72) Erfinder: STYMNE, Sten, S-268 31 Svalöv (SE); LENMAN, Marit, S-268 31 Svalöv (SE); STAHL, Ulf, S-268 31 Svalöv (SE); BANAS, Antoni, S-268 31 Svalöv (SE); CARLSSON, Anders, S-268 31 Svalöv (SE); WIBERG, Eva, S-268 31 Svalöv (SE); DAHLQVIST, Anders, S-268 31 Svalöv (SE)
(74) Vertreter: Fitzner, Uwe
(86) Internationale Anmeldenummer: PCT/EP2003/002955
(87) Internationale Veröffentlichungsnummer: WO 2003/083100

(56) Entgegenhaltungen:
- WO-A-00/60095
- WO-A-01/16308
- VAN DE LOO F.J. ET AL.: "Lipid Metabolism in Plants " 1993 , CRC PRESS INC. , BOCA RATON, FLORIDA, USA XP008020068 ISBN: 0-8493-4907-9 esp.: Table 2; page 106, lines 24-32; page 114, lines 1-16 Seite 91 -Seite 126
- DAHLQVIST A ET AL: "Phospholipid:diacylglycerol acyltransferase: An enzyme that catalyzes the acyl-CoA-independent formation of triacylglycerol in yeast and plants" PROCEEDINGS OF THE NATIONAL ACADEMY OF SCIENCES OF THE UNITED STATES OF AMERICA 06 JUN 2000 UNITED STATES, Bd. 97, Nr. 12, 6. Juni 2000 (2000-06-06), Seiten 6487-6492, XP002235601 ISSN: 0027-8424 in der Anmeldung erwähnt
- ABBADI A ET AL: "Transgenic oilseeds as sustainable source of nutritionally relevant C20 and C22 polyunsaturated fatty acids?" EUROPEAN JOURNAL OF LIPID SCIENCE AND TECHNOLOGY, WILEY VCH VERLAG, WEINHEIM, DE, Bd. 103, Nr. 2, Februar 2001 (2001-02), Seiten 106-113, XP002228744 ISSN: 1438-7697

### SEQUENCE LISTING

<110> BASF Plant Science GmbH
<120> Verwendung eins Enzymgemisches zur Herstellung von pflanzlichen Speicherlipiden enthaltend mehrfach ungesättigte Fettsäuren
<130> 1
<160> 2
<170> PatentIn version 3.1
<210> 1
   <211> 2425
   <212> DNA
   <213> Physcomitrella patens
<220>
   <221> CDS
   <222> (120)..(2135)
   <223> Phospholipid:Diacylglycerin-Acyltransferase
<400> 1
   agaaacagct ctttgtctct ctcgactgat ctaacaatcc ctaatctgtg ttctaaattc
   60
   ctggacgaga tttgacaaag tccgtatagc ttaacctggt ttaatttcaa gtgacagat
   119
   atg ccc ctt att cat cgg aaa aag ccg acg gag aaa cca tcg acg ccg
   167
   Met Pro Leu Ile His Arg Lys Lys Pro Thr Glu Lys Pro Ser Thr Pro
   1               5                   10                  15
   cca tct gaa gag gtg gtg cac gat gag gat tcg caa aag aaa cca cac
   215
   Pro Ser Glu Glu Val Val His Asp Glu Asp Ser Gln Lys Lys Pro His
               20                  25                  30
   gaa tct tcc aaa tcc cac cat aag aaa tcg aac gga gga ggg aag tgg
   263
   Glu Ser Ser Lys Ser His His Lys Lys Ser Asn Gly Gly Gly Lys Trp
           35                  40                  45
   tcg tgc atc gat tct tgt tgt tgg ttc att ggg tgt gtg tgt gta acc
   311
   Ser Cys Ile Asp Ser Cys Cys Trp Phe Ile Gly Cys Val Cys Val Thr
       50                  55                  60
   tgg tgg ttt ctt ctc ttc ctt tac aac gca atg cct gcg agc ttc cct
   359
   Trp Trp Phe Leu Leu Phe Leu Tyr Asn Ala Met Pro Ala Ser Phe Pro
   65 70 75 80
   cag tat gta acg gag cga atc acg ggt cct ttg cct gac ccg ccc ggt
   407
   Gln Tyr Val Thr Glu Arg Ile Thr Gly Pro Leu Pro Asp Pro Pro Gly
   85 90 95
   gtt aag ctc aaa aaa gaa ggt ctt aag gcg aaa cat cct gtt gtc ttc
   455
   Val Lys Leu Lys Lys Glu Gly Leu Lys Ala Lys His Pro Val Val Phe
   100 105 110
   att cct ggg att gtc acc ggt ggg ctc gag ctt tgg gaa ggc aaa caa
   503
   Ile Pro Gly Ile Val Thr Gly Gly Leu Glu Leu Trp Glu Gly Lys Gln
   115 120 125
   tgc gct gat ggt tta ttt aga aaa cgt ttg tgg ggt gga act ttt ggt
   551
   Cys Ala Asp Gly Leu Phe Arg Lys Arg Leu Trp Gly Gly Thr Phe Gly
   130 135 140
   gaa gtc tac aaa agg cct cta tgt tgg gtg gaa cac atg tca ctt gac
   599
   Glu Val Tyr Lys Arg Pro Leu Cys Trp Val Glu His Met Ser Leu Asp
   145 150 155 160
   aat gaa act ggg ttg gat cca gct ggt att aga gtt cga gct gta tca
   647
   Asn Glu Thr Gly Leu Asp Pro Ala Gly Ile Arg Val Arg Ala Val Ser
   165 170 175
   gga ctc gtg gct gct gac tac ttt gct cct ggc tac ttt gtc tgg gca
   695
   Gly Leu Val Ala Ala Asp Tyr Phe Ala Pro Gly Tyr Phe Val Trp Ala
   180 185 190
   gtg ctg att gct aac ctt gca cat att gga tat gaa gag aaa aat atg
   743
   Val Leu Ile Ala Asn Leu Ala His Ile Gly Tyr Glu Glu Lys Asn Met
   195 200 205
   tac atg gct gca tat gac tgg cgg ctt tcg ttt cag aac aca gag gta
   791
   Tyr Met Ala Ala Tyr Asp Trp Arg Leu Ser Phe Gln Asn Thr Glu Val
   210 215 220
   cgt gat cag act ctt agc cgt atg aaa agt aat ata gag ttg atg gtt
   839
   Arg Asp Gln Thr Leu Ser Arg Met Lys Ser Asn Ile Glu Leu Met Val
   225 230 235 240
   tct acc aac ggt gga aaa aaa gca gtt ata gtt ccg cat tcc atg ggg
   887
   Ser Thr Asn Gly Gly Lys Lys Ala Val Ile Val Pro His Ser Met Gly
                   245                 250                 255
   gtc ttg tat ttt cta cat ttt atg aag tgg gtt gag gca cca gct cct
   935
   Val Leu Tyr Phe Leu His Phe Met Lys Trp Val Glu Ala Pro Ala Pro
               260                 265                 270
   ctg ggt ggc ggg ggt ggg cca gat tgg tgt gca aag tat att aag gcg
   983
   Leu Gly Gly Gly Gly Gly Pro Asp Trp Cys Ala Lys Tyr Ile Lys Ala
           275                 280                 285
   gtg atg aac att ggt gga cca ttt ctt ggt gtt cca aaa gct gtt gca
   1031
   Val Met Asn Ile Gly Gly Pro Phe Leu Gly Val Pro Lys Ala Val Ala
       290                 295                 300
   ggg ctt ttc tct gct gaa gca aag gat gtt gca gtt gcc aga gcg att
   1079
   Gly Leu Phe Ser Ala Glu Ala Lys Asp Val Ala Val Ala Arg Ala Ile
   305                 310                 315                 320
   gcc cca gga ttc tta gac acc gat ata ttt aga ctt cag acc ttg cag
   1127
   Ala Pro Gly Phe Leu Asp Thr Asp Ile Phe Arg Leu Gln Thr Leu Gln
                   325                 330                 335
   cat gta atg aga atg aca cgc aca tgg gac tca aca atg tct atg tta
   1175
   His Val Met Arg Met Thr Arg Thr Trp Asp Ser Thr Met Ser Met Leu
               340                 345                 350
   ccg aag gga ggt gac acg ata tgg ggc ggg ctt gat tgg tca ccg gag
   1223
   Pro Lys Gly Gly Asp Thr Ile Trp Gly Gly Leu Asp Trp Ser Pro Glu
           355                 360                 365
   aaa ggc cac acc tgt tgt ggg aaa aag caa aag aac aac gaa act tgt
   1271
   Lys Gly His Thr Cys Cys Gly Lys Lys Gln Lys Asn Asn Glu Thr Cys
       370                 375                 380
   ggt gaa gca ggt gaa aac gga gtt tcc aag aaa agt cct gtt aac tat
   1319
   Gly Glu Ala Gly Glu Asn Gly Val Ser Lys Lys Ser Pro Val Asn Tyr
   385                 390                 395                 400
   gga agg atg ata tct ttt ggg aaa gaa gta gca gag gct gcg cca tct
   1367
   Gly Arg Met Ile Ser Phe Gly Lys Glu Val Ala Glu Ala Ala Pro Ser
                   405                 410                 415
   gag att aat aat att gat ttt cga ggt gct gtc aaa ggt cag agt atc
   1415
   Glu Ile Asn Asn Ile Asp Phe Arg Gly Ala Val Lys Gly Gln Ser Ile
               420                 425                 430
   cca aat cac acc tgt cgt gac gtg tgg aca gag tac cat gac atg gga
   1463
   Pro Asn His Thr Cys Arg Asp Val Trp Thr Glu Tyr His Asp Met Gly
           435                 440                 445
   att gct ggg atc aaa gct atc gct gag tat aag gtc tac act gct ggt
   1511
   Ile Ala Gly Ile Lys Ala Ile Ala Glu Tyr Lys Val Tyr Thr Ala Gly
       450                 455                 460
   gaa gct ata gat cta cta cat tat gtt gct cct aag atg atg gcg cgt
   1559
   Glu Ala Ile Asp Leu Leu His Tyr Val Ala Pro Lys Met Met Ala Arg
   465                 470                 475                 480
   ggt gcc gct cat ttc tct tat gga att gct gat gat ttg gat gac acc
   1607
   Gly Ala Ala His Phe Ser Tyr Gly Ile Ala Asp Asp Leu Asp Asp Thr
                   485                 490                 495
   aag tat caa gat ccc aaa tac tgg tca aat ccg tta gag aca aaa tta
   1655
   Lys Tyr Gln Asp Pro Lys Tyr Trp Ser Asn Pro Leu Glu Thr Lys Leu
               500                 505                 510
   ccg aat gct cct gag atg gaa atc tac tca tta tac gga gtg ggg ata
   1703
   Pro Asn Ala Pro Glu Met Glu Ile Tyr Ser Leu Tyr Gly Val Gly Ile
           515                 520                 525
   cca acg gaa cga gca tac gta tac aag ctt aac cag tct ccc gac agt
   1751
   Pro Thr Glu Arg Ala Tyr Val Tyr Lys Leu Asn Gln Ser Pro Asp Ser
       530                 535                 540
   tgc atc ccc ttt cag ata ttc act tct gct cac gag gag gac gaa gat
   1799
   Cys Ile Pro Phe Gln Ile Phe Thr Ser Ala His Glu Glu Asp Glu Asp
   545                 550                 555                 560
   agc tgt ctg aaa gca gga gtt tac aat gtg gat ggg gat gaa aca gta
   1847
   Ser Cys Leu Lys Ala Gly Val Tyr Asn Val Asp Gly Asp Glu Thr Val
                   565                 570                 575
   ccc gtc cta agt gcc ggg tac atg tgt gca aaa gcg tgg cgt ggc aag
   1895
   Pro Val Leu Ser Ala Gly Tyr Met Cys Ala Lys Ala Trp Arg Gly Lys
               580                 585                 590
   aca aga ttc aac cct tcc gga atc aag act tat ata aga gaa tac aat
   1943
   Thr Arg Phe Asn Pro Ser Gly Ile Lys Thr Tyr Ile Arg Glu Tyr Asn
           595                 600                 605
   cac tct ccg ccg gct aac ctg ttg gaa ggg cgc ggg acg cag agt ggt 1991
   His Ser Pro Pro Ala Asn Leu Leu Glu Gly Arg Gly Thr Gln Ser Gly
   610 615 620
   gcc cat gtt gat atc atg gga aac ttt gct ttg atc gaa gat atc atg 2039
   Ala His Val Asp Ile Met Gly Asn Phe Ala Leu Ile Glu Asp Ile Met
   625 630 635 640
   agg gtt gcc gcc gga ggt aac ggg tct gat ata gga cat gac cag gtc 2087
   Arg Val Ala Ala Gly Gly Asn Gly Ser Asp Ile Gly His Asp Gln Val
   645 650 655
   cac tct ggc ata ttt gaa tgg tcg gag cgt att gac ctg aag ctg tga 2135
   His Ser Gly Ile Phe Glu Trp Ser Glu Arg Ile Asp Leu Lys Leu
   660 665 670
   atatcatgat ctctttaagc tgtcctgtca gcttatgtga atccaatact ttgaaagaga 2195
   gatcatcatc aattcatcat catcgtcatc atcatgatgc tcaactcaca aagaagcctg 2255
   agaatgatac tttggtgcga aattctcaat acctctttaa tattcttatt gaatgtaaat 2315
   tatacaatcc tatctaatgt ttgaacgata acacaaaact tgctgcgcca tgtttgtttg 2375
   tcttgtcaaa agcatcaatt tgtgggttaa aaaaaaaaaa aaaaaaaaaa 2425
<210> 2
   <211> 671
   <212> PRT
   <213> Physcomitrella patens
<400> 2
   Met Pro Leu Ile His Arg Lys Lys Pro Thr Glu Lys Pro Ser Thr Pro
   1 5 10 15
   Pro Ser Glu Glu Val Val His Asp Glu Asp Ser Gln Lys Lys Pro His
   20 25 30
   Glu Ser Ser Lys Ser His His Lys Lys Ser Asn Gly Gly Gly Lys Trp
   35 40 45
   Ser Cys Ile Asp Ser Cys Cys Trp Phe Ile Gly Cys Val Cys Val Thr
       50                  55                  60
   Trp Trp Phe Leu Leu Phe Leu Tyr Asn Ala Met Pro Ala Ser Phe Pro
   65                  70                  75                  80
   Gln Tyr Val Thr Glu Arg Ile Thr Gly Pro Leu Pro Asp Pro Pro Gly
                   85                  90                  95
   Val Lys Leu Lys Lys Glu Gly Leu Lys Ala Lys His Pro Val Val Phe
               100                 105                 110
   Ile Pro Gly Ile Val Thr Gly Gly Leu Glu Leu Trp Glu Gly Lys Gln
           115                 120                 125
   Cys Ala Asp Gly Leu Phe Arg Lys Arg Leu Trp Gly Gly Thr Phe Gly
       130                 135                 140
   Glu Val Tyr Lys Arg Pro Leu Cys Trp Val Glu His Met Ser Leu Asp
   145                 150                 155                 160
   Asn Glu Thr Gly Leu Asp Pro Ala Gly Ile Arg Val Arg Ala Val Ser
                   165                 170                 175
   Gly Leu Val Ala Ala Asp Tyr Phe Ala Pro Gly Tyr Phe Val Trp Ala
               180                 185                 190
   Val Leu Ile Ala Asn Leu Ala His Ile Gly Tyr Glu Glu Lys Asn Met
           195                 200                 205
   Tyr Met Ala Ala Tyr Asp Trp Arg Leu Ser Phe Gln Asn Thr Glu Val
       210                 215                 220
   Arg Asp Gln Thr Leu Ser Arg Met Lys Ser Asn Ile Glu Leu Met Val
   225                 230                 235                 240
   Ser Thr Asn Gly Gly Lys Lys Ala Val Ile Val Pro His Ser Met Gly
                   245                 250                 255
   Val Leu Tyr Phe Leu His Phe Met Lys Trp Val Glu Ala Pro Ala Pro
               260                 265                 270
   Leu Gly Gly Gly Gly Gly Pro Asp Trp Cys Ala Lys Tyr Ile Lys Ala
           275                 280                 285
   Val Met Asn Ile Gly Gly Pro Phe Leu Gly Val Pro Lys Ala Val Ala
       290                 295                 300
   Gly Leu Phe Ser Ala Glu Ala Lys Asp Val Ala Val Ala Arg Ala Ile
   305                 310                 315                 320
   Ala Pro Gly Phe Leu Asp Thr Asp Ile Phe Arg Leu Gln Thr Leu Gln
                   325                 330                 335
   His Val Met Arg Met Thr Arg Thr Trp Asp Ser Thr Met Ser Met Leu
   340 345 350
   Pro Lys Gly Gly Asp Thr Ile Trp Gly Gly Leu Asp Trp Ser Pro Glu
   355 360 365
   Lys Gly His Thr Cys Cys Gly Lys Lys Gln Lys Asn Asn Glu Thr Cys
   370 375 380
   Gly Glu Ala Gly Glu Asn Gly Val Ser Lys Lys Ser Pro Val Asn Tyr
   385 390 395 400
   Gly Arg Met Ile Ser Phe Gly Lys Glu Val Ala Glu Ala Ala Pro Ser
   405 410 415
   Glu Ile Asn Asn Ile Asp Phe Arg Gly Ala Val Lys Gly Gln Ser Ile
   420 425 430
   Pro Asn His Thr Cys Arg Asp Val Trp Thr Glu Tyr His Asp Met Gly
   435 440 445
   Ile Ala Gly Ile Lys Ala Ile Ala Glu Tyr Lys Val Tyr Thr Ala Gly
   450 455 460
   Glu Ala Ile Asp Leu Leu His Tyr Val Ala Pro Lys Met Met Ala Arg
   465 470 475 480
   Gly Ala Ala His Phe Ser Tyr Gly Ile Ala Asp Asp Leu Asp Asp Thr
   485 490 495
   Lys Tyr Gln Asp Pro Lys Tyr Trp Ser Asn Pro Leu Glu Thr Lys Leu
   500 505 510
   Pro Asn Ala Pro Glu Met Glu Ile Tyr Ser Leu Tyr Gly Val Gly Ile
   515 520 525
   Pro Thr Glu Arg Ala Tyr Val Tyr Lys Leu Asn Gln Ser Pro Asp Ser
   530 535 540
   Cys Ile Pro Phe Gln Ile Phe Thr Ser Ala His Glu Glu Asp Glu Asp
   545 550 555 560
   Ser Cys Leu Lys Ala Gly Val Tyr Asn Val Asp Gly Asp Glu Thr Val
   565 570 575
   Pro Val Leu Ser Ala Gly Tyr Met Cys Ala Lys Ala Trp Arg Gly Lys
   580 585 590
   Thr Arg Phe Asn Pro Ser Gly Ile Lys Thr Tyr Ile Arg Glu Tyr Asn
   595 600 605
   His Ser Pro Pro Ala Asn Leu Leu Glu Gly Arg Gly Thr Gln Ser Gly
   610 615 620
   Ala His Val Asp Ile Met Gly Asn Phe Ala Leu Ile Glu Asp Ile Met
   625                 630                 635                 640
   Arg Val Ala Ala Gly Gly Asn Gly Ser Asp Ile Gly His Asp Gln Val
                   645                 650                 655
   His Ser Gly Ile Phe Glu Trp Ser Glu Arg Ile Asp Leu Lys Leu
               660                 665                 670

## Beschreibung

Die vorliegende Erfindung betrifft die Verwendung eines Enzymgemisches enthaltend wenigstens ein Enzym mit Phospholipid:Diacylglycerin-Acyltransferase Aktivität zur Herstellung von pflanzlichen Triacylglyceriden enthaltend mehrfach ungesättigte Fettsäuren.

Triacylglycerid (TAG) stellt den in der Natur am häufigsten vorkommenden Energiespeicher auf Fettbasis dar. Neben Acyl-CoA:Diacylglycerol-Acyltransferasen (DAGAT) sind bislang Phospholipid:Diacylglycerin-Acyltransferasen (PDAT) bekannt, welche die Bildung von Speicherfetten (Triacylglycerid, TAG) katalysieren (Dahlqvist et al., Proc. Natl. Acad. Sci., USA, 2000; 97: 6487-6492). Hierbei katalysieren die Enzyme mit PDAT-Aktivität in einer Acyl-CoA-unabhängigen Reaktion den Transfer von Acylgruppen von der sn-2 Position des Phospholipids auf Diacylglycerin (DAG), wodurch TAG und ein Lyso-Phospholipid gebildet wird.
Biochemische Untersuchungen dieser Transferreaktion wurden bereits in Samen von Ricinus communis und Crepis palestina durchgeführt, die einen hohen Gehalt an Ricinolsäure bzw. Vernolsäure akkumulieren, sowie in Sonnenblumen, die nur gesättigte Fettsäuren in ihrem Saatöl aufweisen, durchgeführt (Dahlqvist et al., Proc. Natl. Acad. Sci., USA, 2000; 97: 6487-6492).

In Pflanzen wie Raps, Sonnenblume, Ölpalme usw. ist das Öl (d.h. Triacylglyceride) das wertvollste Produkt der Samen oder Früchte. Andere Bestandteile wie Stärke, Protein und Faserstoffe werden als Nebenprodukte von geringerem Wert angesehen. Eine Erhöhung der Ölmenge bezogen auf das Gewicht auf Kosten anderer Bestandteile in Ölpflanzen würde daher den Wert der Pflanze erhöhen.

Durch die Veränderung der Aktivität der Gene, welche die Zuteilung von reduziertem Kohlenstoff an die Ölherstellung regulieren, wäre es denkbar, daß die Zellen auf Kosten anderer Produkte mehr Öl akkumulieren. Solche Gene könnten nicht nur in Zellen mit bereits hoher Ölproduktion, wie z. B. Ölpflanzen, verwendet werden, sondern könnten auch eine erhebliche Ölproduktion in Pflanzen mit mäßigem oder niedrigem Ölgehalt, wie z. B. Soja, Hafer, Mais, Kartoffel, Zuckerrüben oder Steckrüben sowie in Mikroorganismen induzieren.

Gene kodierend für eine Phospholipid:Diacylglycerin-Acyltransferase wurden bislang aus Hefe kloniert (WO 00/60095). In Hefe zeigt die PDAT eine Abhängigkeit von den polaren Kopfgruppen des Donorlipids, der transferierten Acylgruppe und der Acylkette des Akzeptormoleküls DAG. Die verstärkte Expression von Hefe-Genen kodierend für Enzyme mit PDAT-Aktivität in dem homologen System der Hefe selbst führt zu einer Erhöhung des Ölgehalts in den jeweiligen Zellen. Dabei werden vor allem einfach ungesättigte Fettsäuren mit Hydroxy-, Epoxy und Acetylengruppen aus der Membran entfernt und in das Speicherlipid TAG überführt (WO 00/60095). Identifizierte ungewöhnliche Fettsäuren wurden beispielsweise von van de Loo et al. klassifiziert ('Lipid Metabolism in Plants' 1993, CRC Press Inc., Boca Raton, Florida, USA, ISBN 0-8493-4907-9).In der WO 00/60095 sind ferner auch zwei Nukleotidsequenzen von Arabidopsis thaliana beschrieben. Durch die Übertragung der Arabidopsis-Gene in Hefe wurde der funktionelle Nachweise erbracht, daß diese Gene aus Arabidopsis für ein Enzym mit PDAT-Aktivität kodieren (WO 00/60095).

Neben einfach ungesättigten Fettsäuren besteht jedoch weltweit insbesondere ein erhebliches Interesse an mehrfach ungesättigten Fettsäuren (Poly-unsaturated Fatty Acids; PUFAs) für den großtechnischen Einsatz. Diese mehrfach ungesättigten Fettsäuren sind beispielsweise zur Ergänzung von Nahrungs- und Futtermitteln von höchstem wirtschaftlichen Interesse. So ist ein hoher Anteil an Lipiden mit ungesättigten Fettsäuren und speziell mit mehrfach ungesättigten Fettsäuren für die Ernährung von Tieren und Menschen wichtig, da diese außerdem einen positiven Einfluss auf den Triglyceridspiegel bzw. Cholesterinspiegel haben und damit das Risiko einer Herzerkrankung reduzieren. Ungesättigte Fettsäuren finden in verschiedenen diätischen Lebensmitteln oder Medikamenten Anwendung. Mehrfach ungesättigte Fettsäuren sind essentielle Nährstoffe, da sie der menschliche und tierische Organismus nicht selbst aufbauen kann. Mehrfach ungesättigte Fettsäuren kommen in der Regel jedoch auch in Pflanzen nicht oder nur in wirtschaftlich uninteressanten Konzentrationen vor.

Aufgabe der vorliegenden Erfindung ist daher, die Bereitstellung mehrfach ungesättigter Fettsäuren aus regenerierbaren Pflanzenrohstoffen, wobei die Nachteile konventioneller Herstellungsmethoden, wie z. B: einer Herstellung durch aufwendige Fermentation aus (mikrobiellen) Einzelzellen, Destillation aus Fischöl oder umweltbelastende Verfahren aus nicht regenerierbaren petrochemischen Erzeugnissen, vermieden werden.

Diese Aufgabe wird gelöst, durch die Verwendung eines Enzymgemisches enthaltend wenigstens ein Enzym mit Phospholipid:Diacylglycerin-Acyltransferase-(PDAT)-Aktivität zur Herstellung von pflanzlichen Triacylglyceriden enthaltend mehrfach ungesättigte Fettsäuren.

Ferner können durch die erfindungsgemäße Verwendung eines Enzymgemisches enthaltend wenigstens ein PDAT-Enzym zusammen mit mindestens einem weiteren Enzym für die Synthese von ungewöhnlichen Fettsäuren, beispielsweise Fettsäuren mit konjugierten Doppelbindungen oder langkettige, mehrfach ungesättigte Fettsäuren, bereitgestellt werden.

Unter "mehrfach ungesättigten Fettsäuren" sind solche Fettsäuren mit einer Kettenlänge von wenigstens 14 C-Atomen zu verstehen, die wenigstens 3 Doppelbindungen aufweisen. Mehrfach ungesättigte Fettsäuren zählen im Sinne der vorliegenden Erfindung zu den ungewöhnlichen Fettsäuren, die in der Regel in Pflanzen nicht vorkommen.

Zu den "ungewöhnlichen Fettsäuren" zählen z. B. Fettsäuren mit Hydroxy-, Epoxy und Acetylengruppen, mehrfach ungesättigte Fettsäuren, bevorzugt langkettige mehrfach ungesättigte Fettsäuren oder Fettsäuren mit konjugierten Doppelbindungen. Interessant sind hierbei zum Beispiel Gamma-Linolensäure, Achachidonsäure, Stearidonsäure, Eicosapentaensäure oder Docosahexaensäure, konjugierte Linolsäure oder konjugierte Linolensäure (CLA). Diese Aufzählung ist jedoch nicht limitierend.

Bevorzugt unter den mehrfach ungesättigten Fettsäuren sind in der vorliegenden Erfindung langkettige mehrfach ungesättigte Fettsäuren. Unter "langkettigen mehrfach ungesättigten Fettsäuren" sind Fettsäuen mit einer Kettenlänge von wenigstens 18 C-Atomen und wenigstens 3 Doppelbindungen zu verstehen. Bevorzugt werden Fettsäuren mit wenigstens 3 Doppelbindungen und mit 18-24, besonders bevorzugt mit 18-22 und insbesondere mit 20 C-Atomen. Hierzu zählen beispielsweise Arachidonsäure, Stearidonsäure, Eicosapentaensäure, Gamma-Linolensäure oder Docosahexaensäure. Bevorzugt wird Arachidonsäure.

Unter konjugierten Fettsäuren sind Fettsäuren mit wenigstens 16 C-Atomen und wenigstens 3 konjugierten Doppelbindungen zu verstehen, wie z. B. CLA (konjugierte Linolensäure).

Bei der Synthese dieser ungewöhnlichen Fettsäuren sind eine Reihe von Enzymen beteiligt, wie z. B. Hydroxylasen, Epoxygenasen, Acetylenasen, Desaturasen, Elongasen, Conjugasen, trans-Desaturasen oder Isomerasen. Die entstehenden ungewöhnlichen Fettsäuren werden von den Pflanzen in die Membranlipide eingebaut.
Da ungewöhnliche Fettsäuren in der pflanzlichen Membran normalerweise nicht vorkommen, muß gewährleistet sein, daß eine korrekte Membranfunktion und somit eine korrekte Zellfunktion gewährleistet bleibt. Aufgrund dessen sollten die ungewöhnlichen Fettsäuren nur in niedriger Konzentration in den Membranlipiden vorliegen. Dies erfordert, daß die ungewöhnlichen Fettsäuren nach ihrem Einbau in die Membranlipide von dort aus wieder effizient entfernt werden. Dies wird in der vorliegenden Erfindung durch den Einsatz eines Enzymgemisches enthaltend wenigstens ein Enzym mit PDAT-Aktivität erreicht, wobei das PDAT-Enzym die ungewöhnlichen Fettsäuren aus den Membranlipiden entfernt und den Triacylglyceriden der Pflanzensamen zuleitet.

Die vorliegende Erfindung umfaßt ferner die Verwendung eines Enzymgemisches enthaltend wenigstens ein Enzym mit Phospholipid:Diacylglycerin-Acyltransferase-Aktivität und wenigstens ein weiteres Enzym mit der Aktivität einer Hydroxylase, Epoxygenase, Acetylenase, Desaturase, Elongase, Conjugase, trans-Desaturasen oder Isomerasen zur Herstellung von pflanzlichen Triacylglyceriden enthaltend mehrfach ungesättigte Fettsäuren.
Die Coexpression von PDAT und weiteren Enzymen, die an der Synthese ungewöhnlicher Fettsäuren beteiligt sind, führt in Pflanzen zu einer höheren Ansammlung ungewöhnlicher Fettsäuren in den Triacylglyceriden, als dies ohne PDAT der Fall ist.

In einer bevorzugten Variante der vorliegenden Erfindung wird ein Enzymgemisch enthaltend ein Enzym mit Phospholipid:Diacylglycerin-Acyltransferase-Aktivität und Desaturase-Aktivität und Elongase-Aktivität verwendet. Diese Verwendung kann so zur Herstellung von Triacylglyceriden enthaltend langkettige mehrfach ungesättigte Fettsäuren dienen. Bevorzugt sind dies Gamma-Linolensäure, Arachidonsäure, Eicosapentaensäure, Stearidonsäure oder Docosahexaensäure.

Erfindungsgemäß umfaßt ist ferner die Verwendung eines Enzymgemisches enthaltend ein Enzym mit Phospholipid:Diacylglycerin-Acyltransferase-Aktivität und Desaturase- oder trans-Desaturase-Aktivität zur Herstellung von Gamma-Linolensäure oder konjugierter Linolsäure. Zur Herstellung von Triacylglyceriden enthaltend konjugierte Fettsäuren, wie z.B. konjugierte Linolensäure (CLA) ist erfindungsgemäß die Verwendung eines Enzymgemisches enthaltend wenigstens ein Enzym mit Phospholipid:Diacylglycerin-Acyltransferase-Aktivität und ein Enzym mit Conjugase-, trans-Desaturase- oder Isomerase-Aktivität, geeignet.

Die Synthese mehrfach ungesättigter Fettsäuren in Pflanzen, deren Einbau in die Membranlipide und die Überführung in Triacylglyceride der Pflanzensamen wird erreicht, indem wenigstens die Aktivität eines PDAT-Enzyms erhöht ist. Dies kann durch eine gesteigerte Genexpression, erhöhte katalytische oder veränderte regulatorische Enyzmaktivität bewerkstelligt werden. Entsprechend erforderliche Maßnahmen sind dem Fachmann bekannt.

Zur Synthese ungewöhnlicher Fettsäuren, wie langkettige mehrfach ungesättigte oder konjugierte Fettsäuren ist erfindungsgemäß die Expression eines Gens kodierend für ein PDAT-Enzym zusammen mit wenigstens einem weiteren Gen kodierend für ein Enzym aus der Gruppe von Hydroxylasen, Epoxygenasen, Acetylenasen, Desaturasen, Elongasen, Conjugasen, trans-Desaturasen oder Isomerasen erforderlich.

Die erhöhte Expression eines Gens kann durch einem Fachmann bekannte Vorgehensweisen erreicht werden. Hierzu zählen beispielsweise die Erhöhung der Kopienzahl des entsprechenden Gens, mindestens um den Faktor 2, vorteilhaft um den Faktor 5-10. Die replizierende Nukleotidsequenz kann dabei erfindungsgemäß chromosomal oder extrachromosomal kodiert vorliegen.

Ferner ist eine operative Verknüpfung mit regulatorischen Sequenzen zu nennen. Sie können die Transkription, die RNA-Stabilität oder das RNA processing sowie die Translation beeinflussen. Beispiele für regulatorische Sequenzen sind u. a. Promotoren, Enhancer, Operatoren, Terminatoren oder Translationsverstärker. Dabei kann es sich um natürliche regulatorische Sequenzen oder modifizierte regulatorische Sequenzen handeln. Auch ist eine Amplifikation regulatorischer Sequenzen denkbar.

Eine gesteigerte Genexpression ist hierbei in Bezug auf eine endogen (natürlich) vorliegende Enzymaktivität zu sehen. Ferner ist im Sinne der vorliegenden Erfindung auch eine heterologe Genexpression umfaßt, also eine Expression von einem oder mehreren Genen, die natürlicherweise nicht in Pflanzen vorkommen, wobei hier eine Steigerung der Genexpression als Steigerung gegenüber einem Nullwert zu sehen ist.

Eine veränderte, bevorzugt erhöhte, katalytische und/oder veränderte regulatorische Aktivität von PDAT-Enzymen oder von weiteren Enzymen, die an der Synthese von ungewöhnlichen Fettsäuren beteiligt sind, können durch gentechnische Veränderungen der entsprechend kodierenden Gensequenz oder durch ein sogenanntes molecular modelling vorgenommen werden. Die hierzu erforderlichen Maßnahmen gehören für den Fachmann zur gängigen Laborpraxis.

Die zuvor gemachten Ausführungen zur verstärkten Expression/Enzymaktivität beziehen sich ebenfalls auf das/die Enzym(e), die zusätzlich zu der Phospholipid:Diacylglycerin-Acyltransferase in einer pflanzlichen Zelle enthalten ist/sind, um in verstärktem Maße ungewöhnliche Fettsäuren in Triacylglyceriden zu überführen.

In einer vorteilhaften Ausgestaltung der vorliegenden Erfindung wird eine Nukleotidsequenz kodierend für ein Enzym mit PDAT-Aktivität verwendet, das aus Pflanzen stammt. Bevorzugt stammt die isolierte Nukleotidsequenz kodierend für ein Enzym mit PDAT-Aktivität aus Arabidopsis thaliana.

In einer weiteren Variante der vorliegenden Erfindung umfaßt das Enzym mit PDAT-Aktivität eine Aminosäuresequenz gemäß SEQ ID No. 2 kodiert durch eine Nukleotidsequenz gemäß SEQ ID No. 1 oder Allele davon.

Diese Sequenzen sind in der WO 00/60095 offenbart als Sequenz mit der Bezeichnung AB006704.

Unter einer isolierten Nukleinsäure oder einem isolierten Nukleinsäurefragment ist erfindungsgemäß ein Polymer aus RNA oder DNA zu verstehen, das einzel- oder doppelsträngig sein kann und optional natürliche, chemisch synthetisierte, modifizierte oder artifizielle Nukleotide enthalten kann. Der Begriff DNA-Polymer schließt hierbei auch genomische DNA, cDNA oder Mischungen davon ein.

Unter Allelen sind erfindungsgemäß funktionell äquivalente, d. h. im wesentlichen gleichwirkende Nukleotidsequenzen zu verstehen. Funktionell äquivalente Sequenzen sind solche Sequenzen, welche trotz abweichender Nukleotidsequenz, beispielsweise durch die Degeneriertheit des genetischen Codes, noch die gewünschten Funktionen besitzen.

Funktionelle Äquivalente umfassen somit natürlich vorkommende Varianten der hierin beschriebenen Sequenzen sowie künstliche, z. B. durch chemische Synthese erhaltene und gegebenenfalls an den Kodon-Gebrauch des Wirtsorganismus angepaßte Nukleotid-Sequenzen. Darüber hinaus umfassen funktionell äquivalente Sequenzen solche, die eine veränderte Nukleotidsequenz aufweisen, welche dem Enzym beispielsweise eine Desensitivität oder Resistenz gegenüber Inhibitoren verleiht.

Unter einem funktionellen Äquivalent versteht man insbesondere auch natürliche oder künstliche Mutationen einer ursprünglich isolierten Sequenz, welche weiterhin die gewünschte Funktion zeigen. Mutationen umfassen Substitutionen, Additionen, Deletionen, Vertauschungen oder Insertionen eines oder mehrerer Nukleotidreste.
Inbegriffen sind hier auch sogenannte Sinnmutationen (sense mutations), die auf Proteinebene beispielsweise zum Austausch konservierter Aminosäuren führen können, welche aber zu keiner grundsätzlichen Veränderung der Aktivität des Proteins führen und somit funktionsneutral sind. Dies beinhaltet auch Veränderungen der Nukleotidsequenz, die auf Proteinebene den N- oder C-Terminus eines Proteins betreffen, ohne jedoch die Funktion des Proteins wesentlich zu beeinträchtigen. Diese Veränderungen können sogar stabilisierenden Einfluß auf die Proteinstruktur ausüben.

Ferner werden beispielsweise auch solche Nukleotidsequenzen durch die vorliegende Erfindung mit umfaßt, welche man durch Modifikation der Nukleotidsequenz, resultierend in entsprechenden Derivaten, erhält. Ziel einer solchen Modifikation kann z. B. die weitere Eingrenzung der darin enthaltenen kodierenden Sequenz oder z. B. auch die Einfügung weiterer Restriktionsenzym-Schnittstellen sein. Funktionelle Äquivalente sind auch solche Varianten, deren Funktion, verglichen mit dem Ausgangsgen bzw. Genfragment, abgeschwächt oder verstärkt ist.

Außerdem sind artifizielle DNA-Sequenzen Gegenstand der vorliegenden Erfindung, solange sie, wie oben beschrieben, die gewünschten Eigenschaften vermitteln. Solche artifiziellen DNA-Sequenzen können beispielsweise durch Rückübersetzung von mittels computergestützten Programmen (molecular modelling) erstellten Proteinen oder durch in-vitro-Selektion ermittelt werden. Besonders geeignet sind kodierende DNA-Sequenzen, die durch Rückübersetzung einer Polypeptidsequenz gemäß der für den Wirtsorganismus spezifischen Kodon-Nutzung erhalten wurden. Die spezifische Kodon-Nutzung kann ein mit molekulargenetischen Methoden vertrauter Fachmann durch Computerauswertungen anderer, bereits bekannter Gene des zu transformierenden Organismus leicht ermitteln.

Unter homologen Sequenzen sind erfindungsgemäß solche zu verstehen, die zu den erfindungsgemäßen Nukleotidsequenzen komplementär sind und/oder mit diesen hybridisieren. Der Begriff hybridisierende Sequenzen schließt erfindungsgemäß substanziell ähnliche Nukleotidsequenzen aus der Gruppe von DNA oder RNA ein, die unter an sich bekannten stringenten Bedingungen eine spezifische Wechselwirkung (Bindung) mit den zuvor genannten Nukleotidsequenzen eingehen. Ein bevorzugtes, nicht einschränkendes Beispiel für stringente Hybridisierungsbedingungen ist die Hybridisierung in 6X Natriumchlorid/Natriumcitrat (SSC) bei etwa 45°C mit anschließendem ein- oder mehrmaligen Waschen in 0,2 X SSC, 0,1% SDS bei 50-65°C. Ferner sind hier kurze Nukleotidsequenzen von z. B. 10 bis 30 Nukleotiden, vorzugsweise 12 bis 15 Nukleotiden, mit eingeschlossen. Primer oder Hybridisierungssonden sind ebenfalls eingeschlossen.

Bei einer homologen Nukleotidsequenz im Rahmen der vorliegenden Erfindung handelt es sich um eine Sequenz, die wenigstens etwa 40%, vorzugsweise wenigstens etwa 50% oder 60%, besonders bevorzugt wenigstens etwa 70%, 80% oder 90% und am meisten bevorzugt wenigstens etwa 95%, 96%, 97%, 98% oder 99% oder mehr Homologie zu einer Nukleotidsequenz gemäß SEQ ID No. 1 aufweist.

Erfindungsgemäß sind auch die den kodierenden Bereichen (Strukturgenen) vorausgehenden (5'- oder upstream) und/oder nachfolgenden (3'- oder downstream) Sequenzbereiche eingeschlossen. Insbesondere sind hierin Sequenzbereiche mit regulatorischer Funktion inbegriffen. Sie können die Transkription, die RNA-Stabilität oder das RNA processing sowie die Translation beeinflussen. Beispiele für regulatorische Sequenzen sind u. a. Promotoren, Enhancer, Operatoren, Terminatoren oder Translationsverstärker.

Gegenstand der vorliegenden Erfindung ist ferner eine Genstruktur enthaltend wenigstens eine Nukleotidsequenz kodierend für eine Phospholipid:Diacylglycerin-Acyltransferase sowie mit dieser operativ verknüpfte regulatorische Sequenzen, welche die Expression der kodierenden Sequenzen in der Wirtszelle steuern.

Geeignete Wirtszellen sind beispielsweise: Pflanzenzellen oder Algenzellen oder Mikroorganismen, wie E. coli, Hefe oder filamentöse Pilze.

Unter einer operativen Verknüpfung versteht man die sequenzielle Anordnung beispielsweise von Promotor, kodierender Sequenz, Terminator und ggf. weiterer regulatorischer Elemente derart, daß jedes der regulatorischen Elemente seine Funktion bei der Expression der kodierenden Sequenz bestimmungsgemäß erfüllen kann. Diese regulatorischen Nukleotidsequenzen können natürlichen Ursprungs sein oder durch chemische Synthese erhalten werden. Als Promotor ist grundsätzlich jeder Promotor geeignet, der die Genexpression in dem entsprechenden Wirtsorganismus steuern kann.
Beispielhaft sei hier der Promotor CaMV35S des Blumenkohlmosaikvirus (Cauliflower Mosaik Virus; Frank et al., 1980, Cell, 21: 285) genannt oder Napin-Promotor aus B. napus (Stalberg et al., 1993, Plant Molecular Biology, 23: 671-683). Ferner kann es sich erfindungsgemäß auch um einen chemisch induzierbaren Promotor handeln, durch den die Expression der ihm unterliegenden Gene in der Wirtszelle zu einem bestimmten Zeitpunkt gesteuert werden kann. Vorteilhaft sind ferner Promotoren, die eine gewebespezfische Expression, bevorzugt Samenspezifische Expression, erlauben. Als Beispiele sind hier folgende Promotoren zu nennen: USP-Promotor (Bäumlein et al., 1991, Mol. Gen. Genet., 225 (3): 459-467), Oleosin-Promotor (WO 98/45461) oder B4-Promotor aus Leguminosen (LeB4; Bäumlein et al., 1992, Plant Journal, 2 (2): 233-239.

Die Herstellung einer Genstruktur erfolgt durch Fusion eines geeigneten Promotors mit wenigstens einer erfindungsgemäßen Nukleotidsequenz nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratury, Cold Spring Harbor, NY (1989) oder Kaiser et al., 1994, Methods in Yeast Genetics, Cold Spring Habor Labroatory Press oder Guthrie et al., 1994, Guide to Yeast Genetics and Molecular Biology, Methods in Enzymology, Academic Press, beschrieben sind.
Für die Verbindung der DNA-Fragmente miteinander können an die Fragmente Adaptoren oder Linker angesetzt werden.

Darüber hinaus betrifft die vorliegende Erfindung einen Vektor enthaltend wenigstens eine Nukleotidsequenz der zuvor beschriebenen Art kodierend für eine Phospholipid:Diacylglycerin-Acyltransferase, mit diesen operativ verknüpfte regulative Nukleotidsequenzen sowie zusätzliche Nukleotidsequenzen zur Selektion transformierter Wirtszellen, für die Replikation innerhalb der Wirtszelle oder zur Integration in das entsprechende Wirtszell-Genom. Ferner kann der erfindungsgemäße Vektor eine Genstruktur der vorgenannten Art enthalten.
Als Vektoren eignen sich beispielsweise solche, die in Mikroorganismen oder Pflanzen repliziert werden. Die nachfolgende Aufzählung ist für die vorliegende Erfindung nicht limitierend: pGEX (Pharmacia Biotech, Inc.; Smith et al., 1988, Gene 67:31-40), pMAL (New England Biolabs, Beverly, MA), pRIT5 (Pharmacia, Piscataway, NJ) mit GlutathionS-Transferase (GTS), Maltose-Bindeprotein oder ProteinA, pTrc(Amann et al., 1988, Gene 69: 301-315), pET-Vektoren (Sudier et al., Genen Expression Technology, Methods in Enzymology 185, Academic Press, San Diego, Californien, 1990: 60-89 und Stratagene, Amsterdam, Niederlande), pYepSec1 (Baldari et al., 1987, Embo J. 6: 229-234), pMFa (Kurjan et al., 1982, Cell 30: 933-943), pJRY88 (Schultz et al., 1987, Gene 54: 113-123), pYES-Derivate (Invitrogen Gorporation, San Diego, CA) oder Vektoren für die Nutzung in filamentösen Pilzen sind beschrieben in: van den Hondel, C.A.M.J.J. & Punt, PI. (1991) "Gene transfer systems and vector development for filamentous fungi, in: Applied Molecular Genetics of Fungi, J.F. Peberdy, et al., eds., p. 1-28, Cambridge University Press: Cambridge. Beispiele für Pflanzenexpressionsvektoren finden sich auch in Becker, D., et al. (1992) "New plant binary vectors with selectable markers located proximal to the left border, Plant Mol Biol 20: 1195-1197 oder in Bevan, MW. (1984) "Binary Agrobacterium vectors for plant transformation", Nucl Acid. Res. 12: 8711-8721. Alternativ können auch Insektenzellexpressionsvektoren genutzt werden z.B. für die Expression in Sf 9 Zellen. Dies sind z.B. die Vektoren der pAc Serie (Smith et al. (1983) Mol Cell Biol 3:2156-2165) und der Vektoren der pVL-Serie (Lucklow and Summers (1989) Virology 170:31-39). Weitere Expressionsvektoren sind pCDM8 und pMT2PC genannt in: Seed, B. (1987) Nature 329:840 oder Kaufman et al., (1987) EMBO J. 31 6: 187-195. Dabei sind vorzugsweise zu nutzende Promotoren viralen Ursprungs wie z.B. Promotoren des Polyoma, Adenovirus 2, Cytomegalovirus oder Simian Virus 40. Weitere prokaryotische und eukaryotische Expressionssysteme sind genannt in Kapitel 16 und 17 in Sambrook et al., Molecular Cloning: A Laboratory Manual 2nd, ed., Cold Spring Harbor Laboratory, Cold Spring Harbor Laboratory Press, Cold Spring Harbor, NY, 1989.

Unter Ausnutzung der erfindungsgemäßen Nukleinsäuresequenzen können entsprechende Sonden oder auch Primer synthetisiert und dazu verwendet werden, beispielsweise mit Hilfe der PCR-Technik analoge Gene aus anderen Organismen zu amplifizieren und isolieren.

Darüber hinaus betrifft die vorliegende Erfindung die aus den Nukleinsäuren abgeleiteten Aminosäuren mit PDAT-Aktivität. Hierzu zählen auch Isoenzyme der PDAT. Unter Isoenzymen versteht man Enzyme, die dieselbe oder eine ähnliche Substratspezifität und/oder katalytische Aktivität, jedoch eine unterschiedliche Primärstruktur aufweisen.
Ferner sind erfindungsgemäß auch modifizierten Formen der PDAT umfaßt. Erfindungsgemäß sind hierunter Enzyme zu verstehen, bei denen Änderungen in der Sequenz, beispielsweise am N- und/oder C-Teminus, des Polypeptids oder im Bereich konservierter Aminosäuren vorliegen, ohne jedoch die Funktion des Enzyms zu beeinträchtigen. Diese Veränderungen können in Form von Aminosäureaustauschen nach an sich bekannten Methoden vorgenommen werden.

Eine besondere Ausführungsvariante der vorliegenden Erfindung umfaßt auch die Verwendung von Varianten der erfindungsgemäßen PDAT, deren Aktivität beispielsweise durch Aminosäureaustausche, verglichen mit dem jeweiligen Ausgangsprotein, abgeschwächt oder verstärkt ist. Gleiches gilt für die Stabilität des erfindungsgemäßen Enzyms in Zellen, die beispielsweise gegenüber dem Abbau durch Proteasen verstärkt oder vermindert anfällig sind.

Ferner sind Polypeptide mit der Funktion einer PDAT Gegenstand der vorliegenden Erfindung, die in ihrer Aminosäuresequenz derart verändert sind, daß sie gegenüber regulatorisch wirkenden Verbindungen, beispielsweise die sie in ihrer Aktivität regulierenden Stoffwechsel-Endprodukte desensitiv sind (feedback-desensitiv).

Die vorliegende Erfindung schließt ferner die Übertragung einer Nukleinsäuresequenz gemäß SEQ ID No. 1 oder eines Teils davon, kodierend für eine PDAT, ein Allel, Homolog oder Derivat davon in ein Wirtssystem ein. Dabei ist auch die Übertragung eines erfindungsgemäßen Genkonstrukts oder Vektors in ein geeignetes Wirtssystem eingeschlossen. Die Übertragung von Fremdgenen in das Genom einer Pflanze wird als Transformation bezeichnet. Es werden dabei allgemein gängige Methoden zur Transformation und Regeneration von Pflanzen aus Pflanzengeweben oder Pflanzenzellen zur transienten oder stabilen Transformation genutzt.
Geeignete Methoden sind die Protoplastentransformation durch Polyethylenglykol-induzierte DNA-Aufnahme, das biolistische Verfahren mit der Genkanone (die sogenannte particle bombardment Methode), die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143, in Potrykus Annu. Rev. Plant Physiol. Plant Molec. Biol. 42, (1991), 205-225, Moloney et al., 1992, Plant Cell Reports, 8: 238-242, Mlynarova et al., 1994, Plant Cell Report., 13: 282-285 sowie Bell et al., 1999, In Vitro Cell. Dev. Biol.-Plant., 35 (6): 456-465 beschrieben.

Vorzugsweise wird das zu exprimierende Konstrukt in einen Vektor kloniert, der geeignet ist, Agrobacterium tumefaciens zu transformieren, beispielsweise pBinl9 (Bevan et al., Nucl. Acids Res. 12 (1984) 8711). Mit einem solchen Vektor transformierte Agrobakterien können dann in bekannter Weise zur Transformation von Pflanzen, insbesondere von Kulturpflanzen, wie z.B. von Tabakpflanzen, verwendet werden, indem beispielsweise verwundete Blätter oder Blattstücke in einer Agrobakterienlösung gebadet und anschließend in geeigneten Medien kultiviert werden. Die Transformation von Pflanzen mit Agrobacterium tumefaciens wird beispielsweise von Höfgen und Willmitzer in Nucl. Acid Res. (1988), 16, 9877 beschrieben oder ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, 5. 15-38.

Mit einem erfindungsgemäßen Vektor transformierte Agrobakterien können ebenfalls in bekannter Weise zur Transformation von Pflanzen wie Testpflanzen wie Arabidopsis oder Kulturpflanzen wie Getreide, Mais, Hafer, Roggen, Gerste, Weizen, Soja, Reis, Baumwolle, Zuckerrübe, Canola, Sonnenblume, Flachs, Hanf, Kartoffel, Tabak, Tomate, Karotte, Paprika, Raps, Tapioka, Maniok, Pfeilwurz, Tagetes, Alfalfa, Salat und den verschiedenen Baum-, Nuß- und Weinspezies, insbesondere von Ölhaltigen Kulturpflanzen, wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne verwendet werden, z.B. indem verwundete Blätter oder Blattstücke in einer Agrobakterienlösung gebadet und anschließend in geeigneten Medien kultiviert werden. Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Ferner umfaßt die vorliegende Erfindung auch Wirtsorganismen, in die wenigstens eine der zuvor genannten Nukleotidsequenzen kodierend für eine Phospholipid:Diacylglycerin-Acyltransferase und/oder ein entsprechendes Genkonstrukt und/oder ein entsprechender Vektor der zuvor genannten Art übertragen wurden. Zusätzlich können die Wirtsorganismen auch noch Nukleotidsequenzen enthalten, die für Enyzme kodieren, die an der Synthese von ungewöhnlichen Fettsäuren beteiligt sind. Auch diese Nukleotidsequenzen können natürlichen Ursprungs oder synthetisch hergestellt sein. Ferner können sie gentechnisch verändert sein und in einem vergleichbaren Genkonstrukt und/oder Vektor der zuvor genannten Art enthalten sein. Dabei ist es denkbar, daß die Nukleotidsequenzen kodierend für eine Phospholipid:Diacylglycerin-Acyltransferase und Enzyme zur Synthese von ungewöhlichen Fettsäuren in einem Genkonstrukt und/oder Vektor enthalten sind oder in verschiedenen Genkonstrukten oder Vektoren vorliegen. In diesen erfindungsgemäßen transgenen Wirtsorganismen, die gegenüber einem entsprechend nicht transformierten Wirtsorganismus eine verstärkte Produktion von ungewöhnlichen Fettsäuren zeigt, liegt die Nukleotidsequenz kodierend für eine Phospholipid:Diacylglycerin-Acyltransferase in erhöhter, wenigstens 2-facher Kopie vor und/oder wird in verstärktem Maße ausgehend von vorgeschalteten regulatorischen Sequenzen exprimiert. Ferner kann ein erfindungsgemäß transformierter Wirtsorganismus gegenüber dem nicht transformierten Wildtyp eine erhöhte Phospholipid:Diacylglycerin-Acyltransferase-Aktivität aufweisen, die einerseits auf einem erhöhten Anteil an in der Zelle vorliegenden Enzymen bzw. u.a. auch aufgrund einer in ihrer katalytischen Aktivität veränderten Phospholipid:Diacylglycerin-Acyltransferase beruht. Ferner kann auch die Regulierung der Enzymaktivität verändert sein.

Erfindungsgemäß handelt es sich bei den Wirtsorganismen prinzipiell um alle Organismen, die in der Lage sind, Fettsäuren und hier speziell ungewöhliche Fettsäuren, wie mehrfach ungesättigte, längerkettige ungesättigte oder konjugierte Fettsäuren, zu synthetisieren oder Organismen, die für die Expression rekombinanter Gene geeignet sind. Hierbei handelt es sich bevorzugt um Pflanzen oder pflanzliche Zellen und bevorzugt um Nutzpflanzen oder deren Zellen. Beispiele für erfindungsgemäß bevorzugte Pflanzen sind Arabidopsis, Asteraceae wie Calendula oder Kulturpflanzen wie Soja, Erdnuß, Rizinus, Sonnenblume, Mais, Baumwolle, Flachs, Leinsamen, Disteln, Raps, Kokosnuß, Ölpalme, Färbersaflor (Carthamus tinctorius) oder Kakaobohne.
Denkbar sind aber auch Mikroorganismen, wie Pilze beispielsweise die Gattung Mortierelia, Saprolegnia oder Pythium, Bakterien, wie die Gattung Escherichia, Hefen, wie die Gattung Saccharomyces, Cyanobakterien, Ciliaten, Algen oder Protozoen, wie Dinoflagellaten oder Crypthecodinium.

Bevorzugt werden Organismen, die natürlicherweise Öle in größeren Mengen synthetisieren können, z. B. Pilze wie Mortierella alpina, Pythium insidiosum oder Pflanzen, wie Soja, Raps, Kokosnuß, Ölpalme, Färbersaflor, Rizinus, Calendula, Erdnuß, Kakaobohne, Sonnenblume oder Hefen, wie Saccharomyces cerevisiae.
Nutzbare Wirtszellen sind weiterhin genannt in: Goeddel, Gene Expression Technology: Methods in Enzymology 185, Academic Press, San Diego, CA (1990).

Die Wirtsorganismen werden in dem Fachmann bekannter Weise angezogen bzw. gezüchtet. Mikroorganismen werden in der Regel in einem flüssigen Medium, das eine Kohlenstoffquelle, meist in Form von Zuckern, eine Stickstoffquelle, meist in Form von organischen Stickstoffquellen, wie Hefeextrakt oder Salzen wie Ammoniumsulfat, Spurenelemente, wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann im batch-, semi- batch- oder kontinuierlichen Ansatz erfolgen. Nährstoffe können zu Beginn der Fermentation vorgelegt oder semikontinuierlich oder kontinuierlich nachgefüttert werden.

Zur Erzeugung transgener Pflanzen werden z. B. binäre Vektoren in Agrobacterium tumefaciens oder Escherichia coli genutzt. Zur Transformation wird eine 1:50 Verdünnung einer Übernachtkultur einer transformierten Agrobakterienkolonie in Murashige-Skoog-Medium (MS-Medium; Murashige and Skoog, 1962, Physiol. Plant, 15: 473) mit 3% Saccharose (3MS-Medium) benutzt. Petiolen oder Hypokotyledonen frisch gekeimter steriler Pflanzen (zu je ca. 1 cm²) werden in einer Petrischale mit einer 1:50 Agrobakterienverdünnung für 5-10 Minuten inkubiert. Es folgt eine 3-tägige Conkubation in Dunkelheit bei 25 °C auf 3MS-Medium mit Bacto-Agar. Die Kultivierung wurde nach 3 Tagen mit 16 Stunden Licht/ 8 Stunden Dunkelheit weitergeführt und in wöchentlichem Rhythmus auf MS-Medium mit Claforan (Cefotaxime-Natrium), Antibiotikum, Benzylaminopurin (BAP) und Glukose weitergeführt. Wachsende Sprosse werden auf MS-Medium mit 2% Saccharose (2MS-Medium), Claforan und Bacto-Agar überführt. Sofern sich keine Wurzeln bilden, wird das Wachstumshormon 2-Indolbuttersäure zum Bewurzeln dem Medium zugesetzt. Regeneriert Sprosse werden auf 2MS-Medium mit Antibiotikum und Claforan erhalten, nach Bewurzelung in Erde überführt und nach Kultivierung für 2 Wochen in eine Klimakammer oder im Gewächshaus angezogen, zur Blüte gebracht, reife Samen geerntet und auf den Fettsäuregehalt analysiert.

Die erfindungsgemäßen transgenen Organismen weisen erfindungsgemäß in ihren Triacylglyceriden einen erhöhten Gehalt an ungewöhnlichen Fettsäuren, wie mehrfach ungesättigte Fettsäuren, lankettige mehrfach ungesättigte Fettsäuren oder konjugierte Fettsäuren, auf. Dabei ist der Gehalt an diesen Fettsäuren im Vergleich zu dem normalerweise in den Triacylglyceriden dieser Pflanzen vorkommenden Fettsäuregehalt erhöht.

Aus den erfindungsgemäß transgenen Organismen werden nach Anzucht die Lipide in üblicherweise gewonnen. Hierzu können die Organismen nach Ernte zunächst aufgeschlossen werden oder direkt verwendet werden. Die Lipide werden vorteilhaft mit geeigneten Lösungsmitteln wie apolare Lösungsmittel wie Hexan oder Ethanol, Isopropanol oder Gemischen wie Hexan/Isopropanol, Phenol/Chloroform/Isoamylalkohol bei Temperaturen zwischen 0 °C bis 80 °C, bevorzugt zwischen 20 °C bis 50 °C extrahiert. Die Biomasse wird in der Regel mit einem Überschuß an Lösungsmittel extrahiert, beispielsweise einem Überschuß von Lösungsmittel zu Biomasse von 1:4. Das Lösungsmittel wird anschließend beispielsweise über eine Destillation entfernt. Die Extraktion kann auch mit superkritischem CO₂ erfolgen. Nach Extraktion kann die restliche Biomasse beispielsweise über Filtration entfernt werden.
Das so gewonnene Rohöl kann anschließend weiter aufgereinigt werden, beispielsweise in dem Trübungen über das Versetzen mit polaren Lösungsmittel wie Aceton oder Chloroform und anschließender Filtration oder Zentrifugation entfernt werden. Auch eine weitere Reinigung über Säulen ist möglich. Zur Gewinnung der freien Fettsäuren aus den Triglyceriden werden diese in üblicher Weise verseift.

Gegenstand der Erfindung sind somit auch ungewöhnliche, mehrfach ungesättigte, längerkettige mehrfach ungesättigte oder konjugierte Fettsäuren sowie Trigylceride mit einem erhöhten Gehalt an diesen Fettsäuren, die nach den oben genannten Vorgehensweisen hergestellt wurden, sowie deren Verwendung zur Herstellung von Nahrungsmitteln, Tierfutter, Kosmetika oder Pharmazeutika. Hierzu werden diese den Nahrungsmitteln, dem Tierfutter, den Kosmetika oder Pharmazeutika in üblichen Mengen zugesetzt.

In einer Variante der vorliegenden Erfindung wurden Arabidopsis thaliana Pflanzen mit der Nukleotidsequenz gemäß SEQ ID No. 1 (AB006704) transformiert. Nachfolgend werden diese Pflanzen hinsichtlich ihrer neuen Eigenschaften analysiert:

### Northern-Blot-Analysen

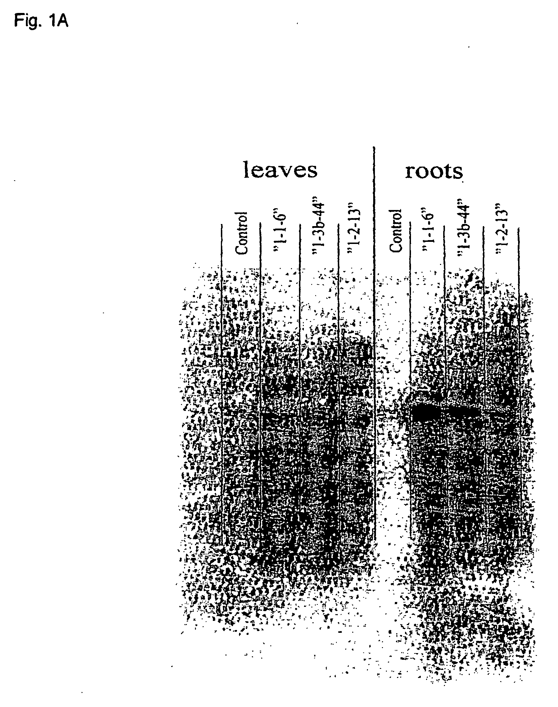

Zur RNA-Extraktion wurden T2 Pflanzen, die mit einem Kontrollvektor oder mit dem Vektor enthaltend das Gen AB006704 transformiert waren, eingesetzt. Da die T2 Keimlinge hinsichtlich des insertierten Gens eine Segregation aufwiesen, wurde Kanamycin zur Elimination von nichttransformierten Keimlingen eingesetzt. T2 Keimlinge von A. thaliana C24 transformiert mit dem Kontrollvektor und T2 Keimlinge von 3 verschiedenen 35S-AB006704 A. thaliana cv. Columbia Transformanten, die nach dem Keimen auf Platten mit Kanamycin überlebt haben, wurden in Flüssigkultur angezüchtet und für die RNA Extraktion benutzt. RNA wurde von Blättern und Wurzeln präpariert und in einen Northern blot aufgetrennt. (Fig. 1a). Die Expression des Arabidopsis AB006704 Gens war den Blättern und Wurzeln von A. thaliana C24 (transformiert mit dem Kontrollvektor) kaum zu detektieren. In allen 3 35S-AB006704 Transformanten war die Expression des AB006704 Gens deutlich, sowohl in Blättern als auch in Wurzeln, sichtbar, wobei die höchste Expression in Wurzeln erfolgte. AB006704 wurde mit der höchsten Expressionsrate in der Transformante Nr. 1-1-6 exprimiert, wobei die Transformaten 1-3b-44 und 1-2-13 Banden mit etwa 70% bzw. 25% der Intensität der Hybridisierungsbande der Transformante 1-1-6 ergaben.

### PDAT Enzymtest

PDAT Aktivität wurde in mikrosomalen Präparationen von Blättern und Wurzeln von T2 Pflanzen von A. thaliana C24 (transformiert mit dem Kontrollvektor) und von T2 Pflanzen von 3 unterschiedlichen 35S-AB006704 Transformanten von A. thaliana cv. Columbia bestimmt. Pflanzen, die für die Mikrosomen-Präparation benutzt wurden, wurden unter den gleichen Bedingungen kultiviert wie Pflanzen, die für die Detektion von AB006704 mRNA durch Northern-Blot-Analysen benutzt wurden. In früheren Experimenten (Dahlqvist et. al., 2000, Proc. Natl. Acas. Sci., USA, 97: 6487-6492; Daten nicht gezeigt) war in den meisten Fällen PC mit Ricinolsäure in Position sn-2 eins der besten Substrate für die durch PDAT katalysierten Reaktionen.

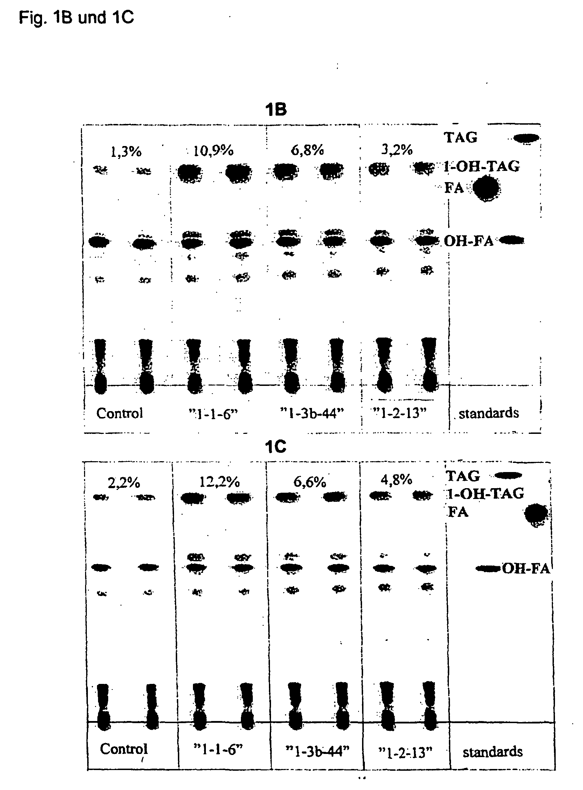

Der Gehalt der de novo synthetisierten 1-OH-TAG gibt im allgemeinen das Expressionsmuster des AB006704 Gens in Pflanzenmaterial wieder, das für die mikrosomale Präparation benutzt wurde (Fig. 1b und 1c). Mikrosomen der Transformante 1-1-6 (höchste AB006704-Genexpression) produzierten mehr TAG als Mikrosomen von Transformante 1-3b-44 (mittleres Expressionsniveau). Die Mikrosomen von Transformante 1-3b-44 synthetisierte mehr TAG als Mikrosomen von Transformante 1-2-13 (niedrige Expression). Ferner produzierten die Mikrosomen der Transformante 1-2-13 mehr TAG als Mikrosomen, die zur Kontrolle mit dem leeren Kontrollvektor transformiert wurde.

### AB006704 kodiert ein Enzym mit PDAT-Aktivität

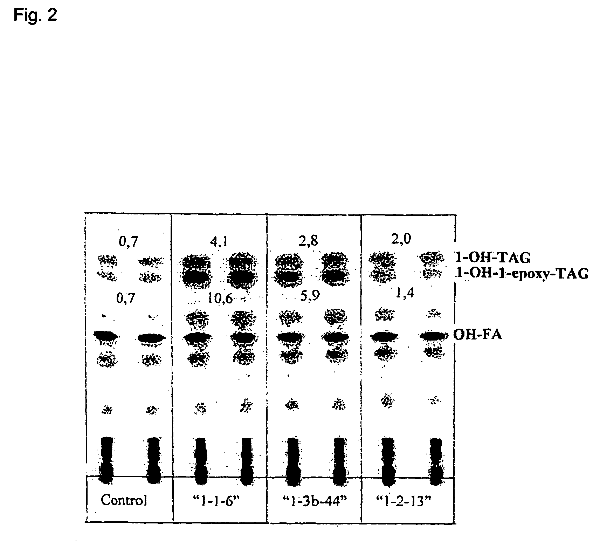

Zum Beweis, daß die Bildung von ¹⁴C-markiertem TAG aus dem vorhergehenden Experiment über eine PDAT-katalysierte Reaktion, mit PC als Donor von Fettsäuren und DAG als Acyl-Akzeptor auftritt, wurde in diesem Experiment sn-1-oleoyl-sn-2-epoxy-DAG als Acyl-Akzeptor eingesetzt und als Acyl-Donor sn-1-oleoyl-sn-[¹⁴C]ricinoleoyl-PC verwendet. Enzymtests wurden mit der gleichen Mikrosomenpräparation von Blättern von T2 Pflanzen von A. thaliana C24 (transformiert mit dem Kontrollvektor) und T2 Pflanzen von 3 verschiedenen Transformanten von A. thaliana cv. Columbia (mit dem AB006704 Gen unter der Kontrolle des 35S Pormotors), wie in dem bereits vorhergehenden Experiment, durchgeführt. Die de novo Synthese von TAG Molekülen enthaltend beide [¹⁴C]-ricinoleoyl und -vernoloyl-Gruppen (Fig. 2) zeigen deutlich die Expressionsmuster des AB006704 Gens in Pflanzenmaterial, welches für die Mikrosomenpräparation (Fig.1a) benutzt wurde. Die erhaltenen Daten zeigen deutlich, daß das transgene PDAT-Gen Fettsäuren von der sn-2 Position von PC auf die Acyl-sn-1-oleoyl-sn-2-epoxy-DAG zur Bildung von TAG nutzen kann. Hierbei werden die Hydroxy- und Epoxy-Fettsäuren in den transgenen Pflanzen enthaltend das Gen AB006704 besser in Triacylglyceride (TAG) eingebaut, als in den Kontrollpflanzen. Aufgrund dessen ist gezeigt, daß das Gen AB006704 ein Enzym mit PDAT-Aktivität kodiert und zur Nutzung von PC als einen intermediären Acyl-Donor und DAG als Acyl-Akzeptor in einer Acyl-CoA-unabhängigen Reaktion zur Bildung von TAG nutzen kann.

### Subtratspezifität

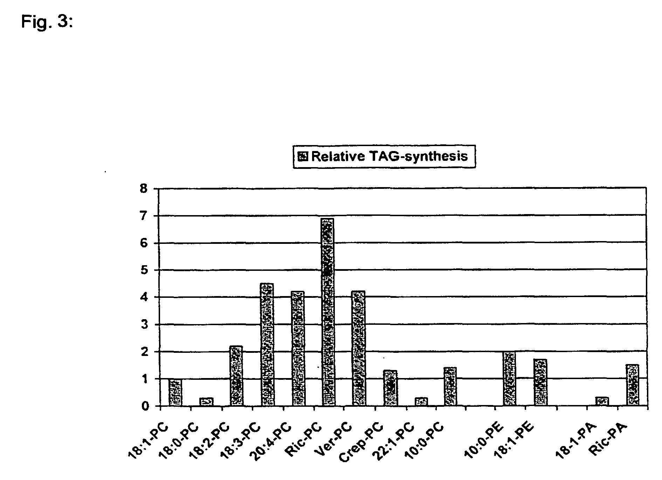

Zur Untersuchung der Substratspezifität (Fig. 3) des A. thaliana Proteins kodiert durch das AB006704-Gen, wurde für den Test eine mikrosomale Präparation von Blättern der Transformante 1-1-6 aus Flüssigkultur genutzt. Das Protein AB006704 zeigte eine höhere Aktivität gegenüber PC mit ungesättigten Fettsäuren in der Position sn-2 als gegenüber PC mit gesättigten Fettsäuren. Unter den 18-C Fettsäuren war PC mit Linolensäure (18:3) in der sn-2 Position das beste Substrat, während Stearinsäure (18:0) am geringsten übertragen wurde. Ferner wurde z.B. Eruicinsäure (22:1) wesentlich schlechter übertragen als Oleinsäure (18:1); Arachidonsäure (20:4) wurde mit annähernd der gleichen Rate wie Linolensäure (18:3) übertragen, obwohl Arachidonsäure eine Doppelbindung mehr als Linolensäure aufweist.
Ricinolsäure, enthaltend eine Hydroxyguppe an Position 12, wurde mit der höchsten Effizienz aller getesteter Acylgruppen auf DAG übertragen. Auch, Vernolsäure, enthaltend eine Epoxygruppe, wurde annähernd doppelt so effizient von der sn-2-Position von PC auf DAG übertragen als die entsprechende Linoleinsäure.
Die untersuchte PDAT aus A. thaliana zeigte außerdem Unterschiede in der Spezifität gegenüber Phospholipiden mit verschiedenen polaren Kopfgruppen. Phosphatidylethanolamin (PE) wurde etwas besser genutzt als Phosphatidylcholin (PC). Die Übertragung von 10:0 bzw. 18:1 von PE war etwa 1,5 mal schneller als von PC. Im Gegensatz dazu war Phosphatidylsäure (PA) ein schlechteres Substrat als PC. Olein- und Ricinolsäure wurden 3- bis 5-mal weniger effizient von Position sn-2 von PA übertragen als von PC. Acyl-Verbindungen des Aktzeptors haben den gleichen Effekt auf die Aktivität der untersuchten PDAT aus A. thaliana. Beispielsweise ist sn-1-oleoyl-sn-2-epoxy-DAG ein etwas besserer Akzeptor als Di-oleoyl-DAG (Fig. 1B und Fig. 2).
Dies bedeutet, daß nicht nur die Kettenlänge, sondern auch die Anzahl der Doppelbindungen sowie ebenfalls die funktionelle Gruppe in der Umgebung der Kopfgruppe die Enzymaktivität beeinflußt.

Nachfolgend wird die vorliegende Erfindung durch weitere Beispiele erläutert, die jedoch nicht limitierend für die Erfindung sind:

### 1. Allgemeine Methoden

Die Isolierung von Plasmid-DNA aus Bakterien oder Pflanzen sowie alle Techniken zur Restriktion, Klenow- und alkalische Phosphatasebehandlung, Elektrophorese, Transformation, Sequenzierung, RNA-Analysen oder PCR wurden nach Sambrook et al. (Molecular cloning. A laboratory manual (1989) Cold Spring Harbour Laboratory Press) durchgeführt.

### 2. Herstellung von Genkonstrukten und Vektoren

Die Nukleotidsequenz AB006704 (SEQ ID No. 1) kodierend für ein PDAT-Enzym aus Arabidopsis thaliana (SEQ ID No. 2) wurde unter Kontrolle des 35S-Promotors gestellt.
Aus dem Ligationsprodukt wurde durch Restriktionsverdau ein Notl-Fragment isoliert und dieses in den binären Vektors pART27 (Lee et al., 1998, Science, 280: 915-918) stromabwärts des Napin-Promotors (zur Samenspezifischen Expression) kloniert. Als Kontrollvektor dient der Vektor pART27 ohne die Nukleotidsequenz kodierend für PDAT aus A. thaliana.

### 3. Transformation von Arabidopsis thaliana

Der zuvor beschriebene Vektor pART27 enthaltend AB006704 wurde in A. thaliana cv. Columbia Pflanzen über Vakuuminfiltration (Bent et. al., 2000, Plant Physiol. 124, (4): 1540-1547) übertragen. Ein entsprechender Kontrollvektor wurde in A. thaliana cv. C24 transformiert. T1 Samen wurde auf 1/3 MS Platten enthaltend 1% Sukrose und 50 µg/ml Kanamycin ausgesät. Gewachsene Keimlinge wurden in Erde ausgepflanzt und T2 Samen geerntet.

Zur Überprüfung, ob das Gen AB006704 das PDAT-Enzym kodiert, wurden Konstrukte zur direkten Expression des AB006704 Gens in Pflanzen hergestellt. Dazu wurde das Gen entweder hinter den CaMV 35S oder den B. napus Napin-Promotor in einen binären Vektor pART27 kloniert und Arabidopsis thaliana (cv. Columbia) durch Vakuuminfiltration transformiert. T2 Keimlinge wurden auf ihre PDAT Aktivität analysiert und die Expression des Gens AB006704 durch Northern Blot Analysen untersucht.

### 4. Mikrosomenpräparation

T2 Samen von A. thaliana cv. C24, transformiert mit dem leeren Vektor und T2 Samen von A. thaliana cv. Columbia, transformiert mit einem Vektor enthaltend AB006704 unter der Kontrolle des 35S-Promotors wurden auf 1/3 MS Platten enthaltend 1% Sucrose und 50 µg/ml Kanamycin ausgesäht. Nicht transformierte A. thaliana Samen (cv. Columbia) wurden auf Platten ohne Kanamycin ausgesäht. Nach 10 Tagen wurden die gewachsenen Keimlinge in Kulturgefäße mit Flüssigkeit enthaltend ½ MS enthaltend 1 % Sucrose übertragen und für 27 Tage bei 23°C unter Licht auf einem Schüttler inkubiert. Mikrosomen von Blättern und Wurzeln der A. thaliana Keimlinge, die in Flüssigkultur gewachsen waren, wurden nach dem Verfahren von Stobart und Stymne (Biochemical Journal, 1985, 232 (1): 217-221) präpariert.

### 5. Herstellung von Lipidsubstraten

Radioaktiv markierte Ricinolsäure (12-Hydroxy-9-octadecensäure) und Vernolsäure (12,13-Epoxy-9-octadecensäure) wurden enzymatisch aus [1-¹⁴C]-Ölsäure bzw. [1-¹⁴C]-Linolsäure durch Inkubation mit Mikrosomenpräparationen aus *Ricinus communis*- bzw. *Crepis palaestina-*Samen synthetisiert (Bafor et al., 1991, Biochem. J. 280: 507-514). Radioaktiv markierte Crepenylsäure (9-Octadeken-12-ynon-Säure) wurde enzymatisch von [1-¹⁴C]-Linolsäure durch Inkubation mit Mikrosomenpräparationen aus Crepis alpina (Lee et al., 1998, Science, 280: 915-918) synthetisiert. Radioaktiv-markierte 10:0, 18:0, 18:1, 18:2, 18:3, 20:1 und 20:4 Fettsäuren sind kommerziell erhältlich. Die Synthese von Phosphatidylcholinen (PC), Phosphatidylethanolaminen (PE) und Phosphatidylsäure (PA) mit ¹⁴C-markierten Acylgruppen in der sn-2-Stellung wurde entweder mit enzymatischer (Banas et al., 1992, Plant Science, 84: 137-144) oder synthetischer Acylierung durchgeführt. Sn-1-oleoyl-sn2-epoxy-DAG wurde von Crepis palaestina Triacylglyceriden durch Lipasebehandlung und Separation über Dünnschichtchromatographie präpariert.

### 6. Enzymtest

Aliquots von Roh-Mikrosomenfraktionen (entsprechend 12 nmol mikrosomalem PC) aus sich entwickelnden Pflanzensamen wurden über Nacht lyophilisiert. ¹⁴C-markierte, in Benzol gelöste Substratlipide (2,5 nmol von PC, PE oder PA mit ¹⁴C-markierten Acylgruppen in der sn-2 Position und 1,5 nmol von Di-oleoyl- oder sn1-oleoyl-sn2-epoxy-DAG) wurden dann zu den getrockneten Mikrosomen gegeben. Das Benzol wurde unter N₂-Begasung verdampft, wodurch die Lipide in direkten Kontakt mit den Membranen gebracht wurden, und 0,1ml 50mM Kaliumphosphat (pH 7,2) wurde zugefügt. Die Suspension wurde gründlich gemischt und bei 30°C über die angegebene Zeitdauer bis zu 60 min inkubiert. Die Lipide wurden aus der Reaktionsmischung mit Chloroform extrahiert und durch Dünnschichtchromatographie unter Verwendung von Kieselgel-60-Platten (Merck) in Hexan/Diethylether/Essigsäure (35:70:1,5 v/v) unter Einsatz von Kieselgel-60-Platten (Merck) getrennt. Die radioaktiven Lipide wurden auf den Platten über elektronische Autoradiographie (Instant Imager, Packard, USA) sichtbar gemacht und quantifiziert.

### 7. Wachstumsexperimente

Nicht transformierte A. thaliana Keimlinge (cv. Columbia) und T2 Keimlinge von A. thaliana (cv. Columbia) transformiert mit dem Vektor enthaltend AB006704 unter der Kontrolle des 35S Promotors (Pflanze 1-1-6 mit dem am höchsten exprimierten Gen AB006704), wurde auf 4 verschiedene 1/3 MS Platten enthaltend 1% Sucrose (die Hälfte der Platten mit Kontrollkeimlingen und die andere Hälfte mit transformierten Keimlingen) ausgesäht und für 17 Tage bei 23°C unter Belichtung kultiviert. Das Frischgewicht von jedem Keimling (ungefähr 50 Keimlinge von jedem Typ) wurde gemessen. Kontrolle und transformierte Keimlinge von jeder Platte wurden gesammelt und für die Lipidanalyse eingesetzt.

### 8. Fettsäuregehalt und Lipidanalyse

Der Fettsäuregehalt in Keimlingen von A. thaliana Wildtyp (cv. Columbia) und entsprechenden Transformanten wurde bestimmt durch die Extraktion von Lipiden nach der Methode von Bligh & Dyer (1959, Can.J. Biochem. Physiol., 37, 911-917) gefolgt von einer Methylierung mit 2% H₂SO₄ in getrocknetem Methanol (60 min. bei 90°C). Lipide in den Arabidopsis Keimlingen (2-3 mg/Probe) wurden direkt methyliert mit 2 ml 2%iger (v/v) H₂SO₄ in getrocknetem Methanol (90 min. bei 90°C). Die Methylester wurden mit Hexan extrahiert und über GLC analysiert unter Einsatz von "Chrompack-Capillarsäulen" (WCOT fused-silica - Säule 50 m x 0,32 mm ID beschichtet CD-Wax 58-CB DF=0,2). Zur Quantifizierung des Fettsäuregehaltes wurde Methylheptadecansäure als interner Standard benutzt.

### Legende zu den Figuren:

Im folgenden wird die vorliegende Erfindung anhand der Figuren noch erläutert.

Figur 1A zeigt eine RNA (Northern-Blot)-Analyse von Gesamt-RNA aus Blättern und Wurzeln von A. thaliana C24 Kontrollpflanzen, die mit dem leeren Kontrollvektor transformiert sind und drei verschiedenen A. thaliana Pflanzen, transformiert mit dem Vektor enthaltend eine Nukleotidsequenz gemäß SEQ ID No. 1 (PDAT) unter der Kontrolle des 35S-Promotors.

Figur 1B und Figur 1C zeigen die Umsetzung von sn-1-oleoyl-sn-2-[¹⁴C]-ricinolyl-PC während einer 1-stündigen Inkubation mit Mikrosomen (und unmarkiertem sn-1-oleoyl-sn-2-oleoly-DAG) aus Blättern (Fig. 1B) und Wurzeln (Fig. 1C) von A. thaliana C24 Kontrollpflanzen und drei verschiedenen A. thaliana Pflanzen, transformiert mit dem Vektor enthaltend eine Nukleotidsequenz gemäß SEQ ID No. 1 (PDAT) unter der Kontrolle des 35S-Promotors. In dem Autoradiogramm ist der durchschnittliche Gehalt an Radioaktivität in dem de-novo synthetisierten 1-OH-TAG als %-Wert angegeben.

Figur 2 zeigt die in-vitro Synthese von TAG enthaltend eine Vernoloyl- und eine [¹⁴C]-ricinoleoyl-Gruppe in Mikrosomen von Blättern von A. thaliana C24 Kontrollpflanze und drei verschiedenen A. thaliana Pflanzen, transformiert mit dem Vektor enthaltend eine Nukleotidsequenz gemäß SEQ ID No. 1 (PDAT) unter der Kontrolle des 35S-Promotors. Die zugegebenen Substrate sind sn-1-oteoyl-sn-2-[¹⁴C]-epoxy-DAG und sn-1-oleoyl-sn-2-[¹⁴C]-ricinoleoyl-PC. In den Autoradiogrammen sind die durchschnittlichen Gehalte an Radioaktität in de-novo synthetisiertem 1-OH-TAG und 1-OH-1-epoxy-TAG angegeben.

Figur 3 zeigt die Substratspezifität des durch die Nukleotidsequenz gemäß SEQ ID No. 1 kodierten Proteins mit PDAT-Aktivität. Es wurden Mikrosomenpräparationen von Blättern der A. thaliana Transformante 1-1-6, transformiert mit dem Vektor enthaltend eine Nukleotidsequenz gemäß SEQ ID No. 1 (PDAT) unter der Kontrolle des 35S-Promotors eingesetzt. Dioleoyl-DAG zusammen mit sn-1-oleoyl-sn-2-[¹⁴C]-Fettsäure-Phospholipiden (PC, PE, PA) wurden als Substrate (18:0-PC, 20:4-PC, 22:1-PC, 10:0-PC und 10:0-PE mit 16:0 in Position sn-1) eingesetzt. Relative Enzymaktivitäten der PADAT gegenüber verschiedenen Substraten sind dargestellt, wobei die Aktivität gegenüber 18:1-PC als 1 festgesetzt wurde. (20:4 ist Arachidonsäure).

Ferner ist ein Sequenzprotokoll enthaltend SEQ ID No. 1 und SEQ ID No. 2 für ein PDAT-Enzym aus Arabidopsis thaliana angefügt.

## Patentansprüche

1. Verwendung eines Enzymgemisches enthaltend wenigstens ein Enzym mit Phospholipid:Diacylglycerin-Acyltransferase-Aktivität zur Herstellung von pflanzlichen Triacylglyceriden enthaltend mehrfach ungesättigte Fettsäuren.

2. Verwendung gemäß Anspruch 1, wobei ein Enzymgemisch enthaltend wenigstens ein Enzym mit Phospholipid:Diacylglycerin-Acyltransferase-Aktivität und wenigstens ein weiteres Enzym mit der Aktivität einer Hydroxylase, Epoxygenase, Acetylenase, Desaturase, Elongase, Conjugase, trans-Desaturase oder Isomerase eingesetzt wird.

3. Verwendung gemäß einem der Ansprüche 1 oder 2, wobei ein Enzymgemisch enthaltend ein Enzym mit Phospholipid:Diacylglycerin-Acyltransferase-Aktivität, Desaturase- und Elongase-Aktivität eingesetzt wird.

4. Verwendung gemäß einem der Ansprüche 1-3 zur Herstellung von Triacylglyceriden enthaltend langkettige mehrfach ungesättigte Fettsäuren.

5. Verwendung gemäß einem der Ansprüche 1-4 zur Herstellung von Triacylclyceriden enthaltend Gamma-Linolensäure, Arachidonsäure, Eicosapentaensäure, Stearidonsäure oder Docosahexaensäure.

6. Verwendung gemäß einem der Ansprüche 1-5, wobei das Enzym mit PDAT-Aktivität durch eine replizierbare Nukleotidsequenz kodiert wird, welche in wenigstens 2-facher Kopie in einer pflanzlichen Zelle vorliegt und/oder welche regulatorische Sequenzen enthält, die eine wenigstens 2-fache Erhöhung der Genexpression und/oder Aktivität des Enzyms mit PDAT-Aktivität bewirkt.

7. Verwendung gemäß einem der Ansprüche 1 bis 6, wobei die replizierende Nukleotidsequenz kodierend für ein Enzym mit PDAT-Aktivität chromosomal oder extrachromosomal kodiert vorliegt.

8. Verwendung gemäß einem der Ansprüche 1 bis 6, wobei die Nukleotidsequenz kodierend für ein Enzym mit PDAT-Aktivität aus Pflanzen stammt.

9. Verwendung gemäß einem der Ansprüche 1 bis 6, wobei die Nukleotidsequenz kodierend für ein Enzym mit PDAT-Aktivität aus Arabidopsis thaliana stammt.

10. Verwendung gemäß einem der Ansprüche 1 bis 6, wobei das Enzym mit PDAT-Aktivität eine Aminosäuresequenz gemäß SEQ ID No. 2 kodiert durch eine Nukleotidsequenz gemäß SEQ ID No. 1 oder Allele davon umfaßt.

## Claims

1. The use of an enzyme mixture comprising at least one enzyme with phospholipid:diacylglycerol acyltransferase activity for the production of plant triacyl glycerides comprising polyunsaturated fatty acids.

2. The use according to claim 1, wherein an enzyme mixture comprising at least one enzyme with phospholipid:diacylglycerol acyltransferase activity and at least one further enzyme with the activity of a hydroxylase, epoxygenase, acetylenase, desaturase, elongase, conjugase, trans-desaturase or isomerase is employed.

3. The use according to claim 1 or 2, wherein an enzyme mixture comprising an enzyme with phospholipid:diacylglycerol acyltransferase activity, desaturase and elongase activity is employed.

4. The use according to any of claims 1-3 for the production of triacyl glycerides comprising long-chain polyunsaturated fatty acids.

5. The use according to any of claims 1-4 for the production of triacyl glycerides comprising gamma-linolenic acid, arachidonic acid, eicosapentaenoic acid, stearidonic acid or docosahexaenoic acid.

6. The use according to any of claims 1-5, wherein the enzyme with PDAT activity is encoded by a nucleotide sequence which is capable of replication, is present in a plant cell in at least 2 copies and/or comprises regulatory sequences bringing about an at least 2-fold increase in gene expression and/or activity of the enzyme with PDAT activity.

7. The use according to any of claims 1 to 6, wherein the replicating nucleotide sequence encoding an enzyme with PDAT activity is encoded chromosomally or extrachromosomally.

8. The use according to any of claims 1 to 6, wherein the nucleotide sequence encoding an enzyme with PDAT activity is derived from plants.

9. The use according to any of claims 1 to 6, wherein the nucleotide sequence encoding an enzyme with PDAT activity is derived from Arabidopsis thaliana.

10. The use according to any of claims 1 to 6, wherein the enzyme with PDAT activity comprises an amino acid sequence as shown in SEQ ID No. 2 encoded by a nucleotide sequence as shown in SEQ ID No. 1 or alleles thereof.

## Revendications

1. Utilisation d'un mélange d'enzymes contenant au moins une enzyme ayant une activité de phospholipide:diacylglycérol-acyltransférase pour préparer des triacylglycérides végétaux contenant des acides gras polyinsaturés.

2. Utilisation selon la revendication 1, dans laquelle on utilise un mélange d'enzymes contenant au moins une enzyme ayant une activité de phospholipide:diacylglycérol-acyltransférase et au moins une autre enzyme ayant l'activité d'une hydroxylase, d'une époxygénase, d'une acétylénase, d'une désaturase, d'une élongase, d'une conjugase, d'une trans-désaturase ou d'une isomérase.

3. Utilisation selon l'une des revendications 1 ou 2, dans laquelle on utilise un mélange d'enzymes contenant une enzyme ayant une activité de phospholipide:diacylglycérol-acyltransférase, une activité de désaturase et d'élongase.

4. Utilisation selon l'une des revendications 1 à 3 pour préparer des triacylglycérides contenant des acides gras polyinsaturés à longue chaîne.

5. Utilisation selon l'une des revendications 1 à 4 pour préparer des triacylglycérides contenant de l'acide gamma-linolénique, de l'acide arachidonique, de l'acide éicosapentaénoïque, de l'acide stéaridonique ou de l'acide docosahexaénoïque.

6. Utilisation selon l'une des revendications 1 à 5, dans laquelle l'enzyme ayant une activité de PDAT est codée par une séquence nucléotidique réplicable, qui est présente en une copie au moins double dans une cellule végétale et/ou qui contient des séquences régulatrices qui provoquent une multiplication au moins par deux de l'expression génique et/ou de l'activité de l'enzyme ayant une activité de PDAT.

7. Utilisation selon l'une des revendications 1 à 6, dans laquelle la séquence nucléotidique qui se réplique, codant pour une enzyme ayant une activité de PDAT, se présente sous forme à codage chromosomique ou extrachromosomique.

8. Utilisation selon l'une des revendications 1 à 6, dans laquelle la séquence nucléotidique codant pour une enzyme ayant une activité de PDAT dérive de végétaux.

9. Utilisation selon l'une des revendications 1 à 6, dans laquelle la séquence nucléotidique codant pour une enzyme ayant une activité de PDAT dérive d'Arabidopsis thaliana.

10. Utilisation selon l'une des revendications 1 à 6, dans laquelle l'enzyme ayant une activité de PDAT comprend une séquence d'acides aminés selon SEQ ID N° 2 codée par une séquence nucléotidique selon SEQ ID N° 1, ou des allèles de cela.
